# EUROPEAN PATENT APPLICATION

(11) **EP 3 590 937 A1**
(43) Date of publication of application: **08.01.2020**
(21) Application number: 18760371.7
(22) Date of filing: 02.03.2018
(51) Int. Cl.: C07D 413/12, C07D 271/08, G01N 30/06, G01N 30/88

(54) **NOVEL COMPOUND, FLUORESCENCE DERIVATIZATION REAGENT INCLUDING SAID NOVEL COMPOUND, METHOD FOR OPTICALLY RESOLVING OPTICAL ISOMER OF AMINO ACID IN WHICH SAID NOVEL COMPOUND IS USED, AND FLUORESCENCE DERIVATIZED AMINO ACID**

(30) Priority: 03.03.2017 JP 2017041128
(71) Applicant: Shiseido Company Ltd., Chuo-ku Tokyo 104-0061 (JP)
(72) Inventor: HAMASE, Kenji, Fukuoka-shi Fukuoka 812-8581 (JP); AKITA, Takeyuki, Fukuoka-shi Fukuoka 812-8581 (JP); MITA, Masashi, Tokyo 104-0061 (JP)
(74) Representative: Scholz, Volker
(86) International application number: PCT/JP2018/008154
(87) International publication number: WO 2018/159841

(57) **Abstract**

The object of the present invention is to develop a reagent for optical resolution for the analysis of chiral amino acids wherein quenching is not exhibited. This object is achieved by providing a novel compound for optical resolution wherein quenching is not exhibited. The present invention relates to a novel compound, a reagent for optical resolution comprising the novel compound, a method for optically resolution comprising a step of reacting the novel compound, and optical isomers obtained by reacting the novel compound with amino acids.

## Description

### FIELD

The present invention relates to a novel compound, a regent for fluorescence derivatization comprising the novel compound, a method for optically resolving fluorescently derivatized amino acids obtained by reacting the novel compound with amino acids, and the fluorescently derivatized amino acids obtained by reacting the novel compound with amino acids.

### BACKGROUND

Conventionally, amino acids present *in vivo* in higher animals were thought to be only L-amino acids. However, recently, due to advances in analytical techniques, it has become clear that D-amino acids are also present *in vivo* in higher animals, particularly in mammals including humans, and are involved in physiological functions such as memory and learning as well as hormone production and secretion. Furthermore, some of the D-amino acids present in various tissues and biological samples have been shown to fluctuate depending on the condition of the body, for example, due to disorders such as kidney failure. Thus, the use thereof as health and disease markers are anticipated (Patent Literature 1).

For the purpose of elucidating the physiological roles of amino acids *in vivo,* advances are being made in developmental research into analytical techniques that accurately separate and quantify amino acids (e.g. Non-Patent Literature 1 to 3) or D-amino acids and L-amino acids (e.g. refer to non-patent document 4) using high-performance liquid chromatography (HPLC). Furthermore, a method for selectively analyzing all the amino acids constituting a protein comprising fluorescence derivatization using 4-fluoro-7-nitro-2,1,3-benzoxadiazole (NBD-F), which is a conventional labelling reagent for HPLC, combined with a two-dimensional chiral HPLC has been developed (Non-Patent Literature 5). Furthermore, reagents for optical resolution that can optically resolve mixtures of optical isomers of amino acids more simply and sensitively have been developed (Patent Literature 2).

### [CITATION LIST]

### [PATENT LITERATURE]

[PTL 1] WO2013/140785
[PTL 2] Japanese Unexamined Patent Publication (Kokai) No. 2015-48332

### [NON PATENT LITERATURE]

[NPL 1] K. Shimbo et al., Anal. Chem, 2009, 81, 5172-5179
[NPL 2] K. Shimbo et al., Rapid Commun Mass Spectrom., 2009, 23, 1483-1492
[NPL 3] S. A. Cohen et al., Anal. Biochem., 1993, 211,279-287
[NPL 4] R. J. Reischl et al., J. Chromatogra. A., 2012, 1269, 262-269
[NPL 5] T. Kimura et al., Scientific Reports, (2016), 6, 26137

### SUMMARY

### [TECHNICAL PROBLEM]

In a D-amino acid analysis technique using the conventional HPLC labelling reagent 4-fluoro-7-nitro-2,1,3-benzoxadiazole (NBD-F), a problem was discovered wherein quenching occurred in NBD-tryptophan produced by a reaction of tryptophan with the NBD-F reagent whereby the detection sensitivity decreased. Typical amino acid analysis systems have a measurement range of 3 to 4 orders of magnitude. However, the measurement values of NBD-tryptophan is not within such a range due to quenching, making simultaneous analysis with at least 40 other chiral amino acids difficult and as such the reagent could not be used in a metabolomic technique to comprehensively analyze metabolites.

### [SOLUTION TO PROBLEM]

In order to solve the aforementioned problem, a compound which does not cause quenching at the time of derivatizing amino acids was screened. Then, it was discovered that by derivatizing tryptophan using the compound according to the following formula, the obtained tryptophan derivative did not exhibit quenching and the D-amino acid and the L-amino acid of the tryptophan derivative could be separated by chiral column chromatography, and thus the present invention was achieved.

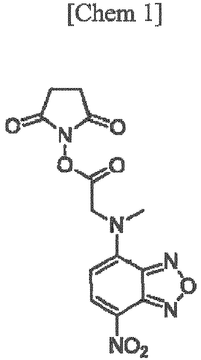

Specifically, the present invention relates to the following:
[1] A compound according to the following formula: 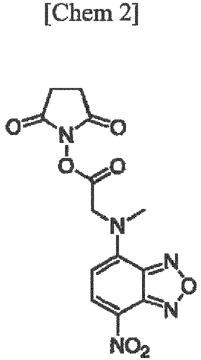 or a salt thereof.
[2] A fluorescence derivatization reagent comprising a compound according to the following formula: 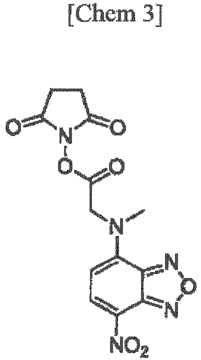 or a salt thereof.
[3] A method of analyzing an amino group-containing compound in a sample comprising:
   reacting the sample comprising the amino group-containing compound with a compound according to the following formula: 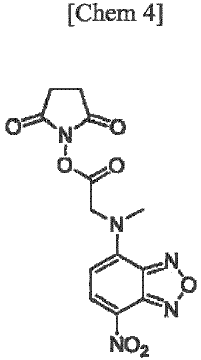 ; and
   subjecting a reaction product to chromatography.
[4] The method according to [3] wherein the method of analysis is a method of optically resolving optical isomers and the chromatography is chiral chromatography.
[5] The method according to [3] and [4] wherein the amino group-containing compound is an amino acid.
[6] A method for producing a fluorescent amino acid derivative comprising reacting an amino acid with a compound according to the following formula: 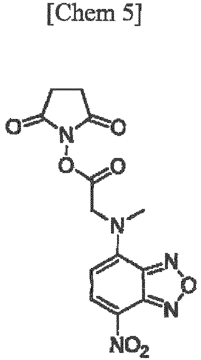
[7] A compound according to the following formula: 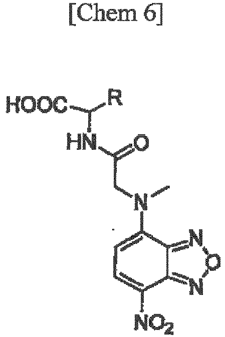 wherein R represents an amino acid side chain.

### [ADVANTAGEOUS EFFECTS OF INVENTION]

According to the present invention, a compound for fluorescence derivatization used in optical resolution is provided that can suppress a decrease in detection sensitivity due to quenching.

### BRIEF DESCRIPTION OF DRAWINGS

[FIG. 1] is a chromatogram obtained by subjecting a sample comprising proteinogenic amino acids to fluorescence derivatization with NBD-F and then to HPLC analysis.
[FIG. 2] shows chromatograms obtained by derivatizing a sample containing D-alanine and L-alanine, and D-tryptophan and L-tryptophan with (A) NBD-F or (B) and (C) NBD-COOSu, and then subjecting them to chiral HPLC analysis. Chiral HPLC separated products are measured by fluorescence analysis in (A) and (B), while the separated products of chiral HPLC are measured by mass analysis in in (C).

### DESCRIPTION OF EMBODIMENTS

### (Novel compound)

The inventors of the present invention newly produced the compound (2,5-dioxopyrrolidin-1-yl 2-[N-(4-nitrobenzo [c] [1,2,5] oxadiazol-7-yl)-N-methylamino] acetate (NBD-COOSu)) as represented by the following formula. Accordingly, one embodiment of the present invention relates to the compound according to the following formula: 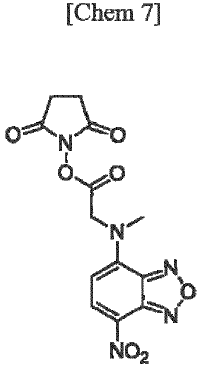 or a salt thereof. The salt may include those formed with any acid, for example, inorganic acid salts such as hydrochlorides, hydrobromides, phosphates, sulfates and perchlorates, and organic acid salts such as acetates, oxalates, maleates, tartrates, citrates, succinates, benzoates and malonates.

### (Synthesis of novel compound)

This compound can be produced by any method and may, for example, be produced based on the following scheme 1 and scheme 2.

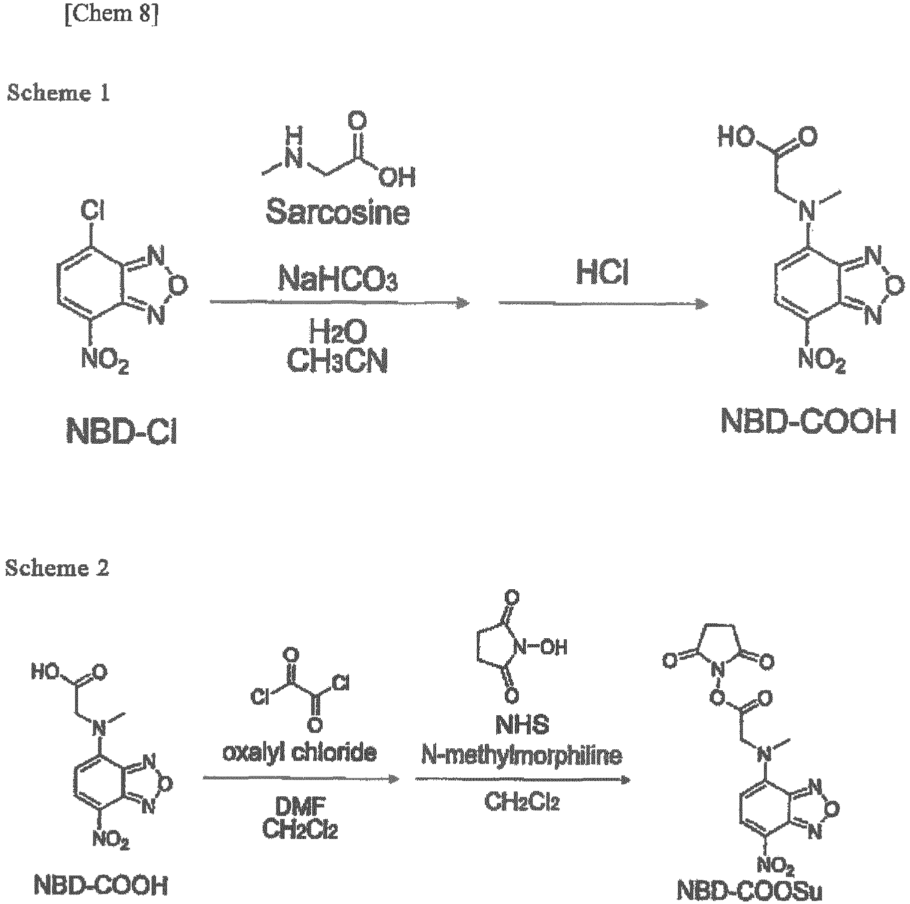

### (Reagent for fluorescence derivatization and reaction thereof)

The compound of the present invention emits fluorescence based on nitrobenzoxadiazole (NBD) and reacts with a compound having an amino group based on the reactivity of a succinimidyl group to form a fluorescent derivative. The compound of the present invention can react with any compound having an amino group, for example, an amino acid. Specifically, amino acid fluorescent derivatives are produced based on, for example, the following scheme. Accordingly, another embodiment of the present invention relates to a reagent for fluorescence derivatization comprising 2,5-dioxopyrrolidin-1-yl 2-[N-(4-nitrobenzo [c] [1,2,5] oxadiazol-7-yl) -N-methylamino] acetate (NBD-COOSu). As will be described later, the D-amino acid can be separated from the L-amino acid by subjecting amino acids which are fluorescently derivatized with a fluorescence derivatization reagent to chiral chromatography. Thus, the fluorescence derivatization reagent of the present invention can be referred to as an optical resolution reagent. 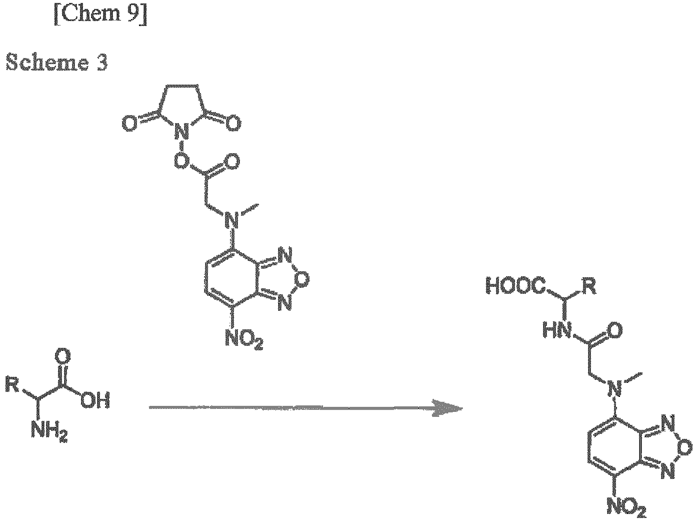 wherein R represents any amino acid side chain.

In another embodiment, the present invention relates to a method for producing an amino acid fluorescence derivative comprising a step of reacting the compound of the present invention with an amino acid. Further, the present invention also relates to a fluorescent derivative of an amino acid produced by the production method, namely, a fluorescent amino acid derivative having the following formula: 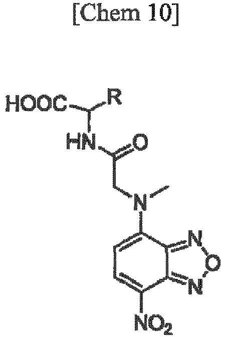 wherein R represents any amino acid side chain.

In the present invention, R represents any amino acid side chain and may be a side chain of a proteinogenic amino acid. Furthermore, from the standpoint of optical resolution, any proteinogenic amino acid side chains excluding the glycine side chain (-H) are suitable. Typical examples thereof include the alanine side chain (-CH₃) and a tryptophan side chain according to the following formula.

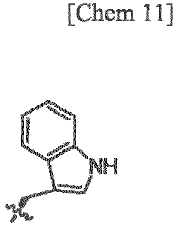

These fluorescent amino acid derivatives can emit fluorescence which arises from the NBD part and are characterized in that fluorescence quenching that can arise due to the structure of some amino acid side chains does not occur. Furthermore, these fluorescent amino acid derivatives retain the chirality of the original amino acid and are thus also characterized in that the D-amino acids and L-amino acids can be separated based on the chirality of the original amino acids by subjecting the fluorescent amino acid derivatives to chiral chromatography.

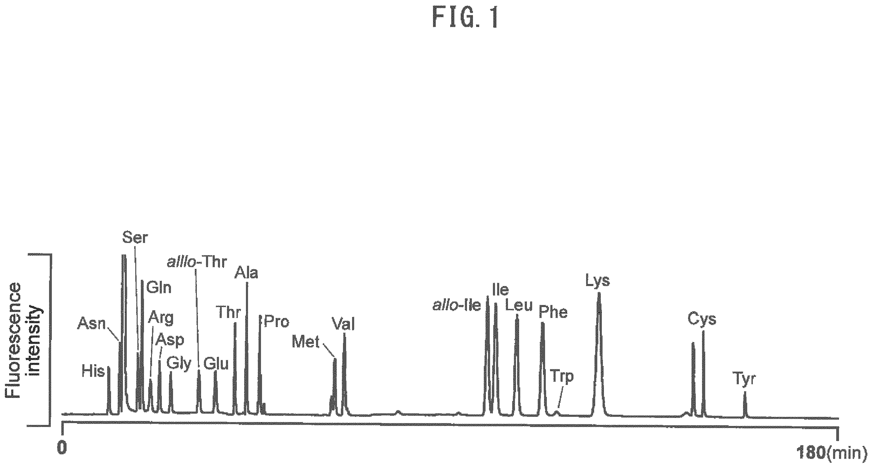

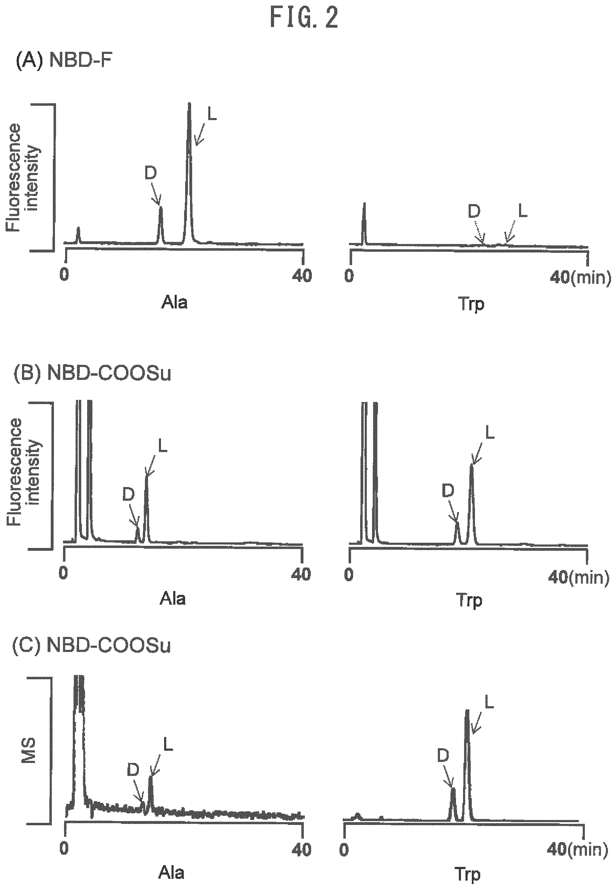

When derivatization was carried out using conventional NBD-F, the tryptophan derivative exhibited quenching (FIG. 1 and FIG. 2A). Thus, tryptophan derivatives that do not exhibit quenching are particularly useful for the comprehensive analysis of D-amino acids. It is clear from the examples of the present invention that the tryptophan derivative from the aforementioned amino acid derivatives does not exhibit quenching (FIG. 2B). Although it is not intended to be bound by theory, quenching herein refers to the loss of fluorescence due to a structure in a compound, which is in adjacent to the structure having fluorescence.

By performing optical resolution chromatography on fluorescent derivatives of amino acids which is obtained by reacting the compound according to the present invention with compounds that have optical isomers, for example, amino acids (D-amino acid + L-amino acids), it make it possible to separate and analyze the fluorescent derivatives based on the original optical isomer. Thus, yet another embodiment of the present invention relates to a reagent for optical resolution comprising the compound of the present invention, and may relate to a kit or system for optical resolution and/or fluorescence derivatization comprising a reagent for optical resolution and/or fluorescence derivatization, and an optical resolution column.

In another embodiment of the present invention, the present invention relates to a method for analyzing an amino group-containing compound in a sample. The method specifically comprises:
reacting a sample comprising an amino group-containing compound with a compound according to the following formula: 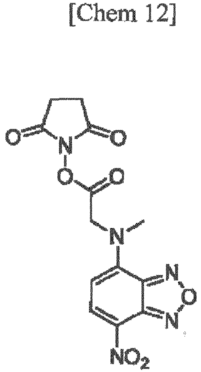 , and
subjecting the reaction product to chromatography. The analysis method according to the present invention is preferably an optical resolution method for separating especially optical isomers. In such cases, chiral chromatography is performed as the chromatography. Any compounds having an amino group, for example amino acids, can be used. Amino acids, particularly optical isomers of proteinogenic amino acids, namely D-amino acids and L-amino acids can be separated and analyzed.

In the step of reacting a sample comprising an amino group-containing compound with the compound of the present invention, the sample comprising an amino group-containing compound is mixed in a solution containing the compound of the present invention and the reaction can be performed while heating if necessary. This reaction is preferably performed in the dark in order to avoid fluorescence fading. An appropriate solvent can be optionally used, for example, water, methanol, ethanol, acetonitrile, etc. may be used.

The sample comprising an amino group-containing compound may be any of, for example, biological samples, environmental samples, experimental samples, and industrial samples (food samples, pharmaceutical samples, cosmetic samples). Biological samples may include: body fluid, such as blood, saliva, tears, or lymph; tissue samples such as cells or organs; and excreta samples such as urine and feces. These samples may be directly reacted with the compound of the present invention but are preferably purified appropriately according to the type of sample. Further, purification can be performed after the reaction step. The method of purifying the sample can be performed by selecting filtering, deproteinization, solvent extraction, ion exchange, solid phase extraction, and the like or by combining thereof, according to the type of sample.

In the aforementioned reaction step, the amino group-containing compound derivatized by reacting with the compound of the present invention is subjected to a chromatography equipped with an optical resolution column. The type of chromatography is preferably HPLC. Before subjecting a compound to the chromatography equipped with the optical resolution column, pre-chromatography using a different column system may be carried out. For the optical resolution column, any column can be selected that separates D-amino acids and L-amino acids derivatized by the compound of the present invention. The optical resolution column comprises a solid phase consisting of a compound comprising just one of the optical isomers. For example, glycine derivatives, leucine derivatives, and quinine derivatives may be used. The amino acid derivatives separated into D-amino acids and L-amino acids by chromatography can be analyzed by detecting the fluorescence thereof. The detection of fluorescence can be carried out by a detector included in the chromatography system. The detector irradiates the compound with light at the excitation wavelength (470 nm) which excites NBD and detects fluorescence derived from excited NBD (fluorescence wavelength of 530 nm) whereby derivatized amino acids can be analyzed.

Proteinogenic amino acids refer to the 20 amino acids that constitute protein. The proteinogenic amino acids are alanine (Ala/A), arginine (Arg/R), asparagine (Asn/N), aspartic acid (Asp/D), cysteine (Cys/C), glutamic acid (Glu/E), glutamine (Gln/Q), glycine (Gly/G), histidine (His/H), isoleucine (Ile/I), leucine (Leu/L), lysine (Lys/K), methionine (Met/M), phenylalanine (Phe/F), proline (Pro/P), serine (Ser/S), threonine (Thr/T), tryptophan (Trp/W), tyrosine (Tyr/Y), and valine (Val/V). The optically isomeric D-amino acid and L-amino acid forms exist for these amino acids excluding glycine. Furthermore, threonine and isoleucine have allo forms. In the method of analyzing amino acids in a sample by optical resolution according to the present invention, the amino acids to be analyzed are preferably proteinogenic amino acids other than glycine, but other amino acids such as ornithine or citrulline may be analyzed.

### EXAMPLES

### EXAMPLE 1: Synthesis of 2,5-dioxopyrrolidin-1-yl 2-[N-(4-nitrobenzo [c] [1,2,5] oxadiazol-7-yl)-N-methylamino] acetate (NBD-COOSu)

### (1) Synthesis of NBD-COOH

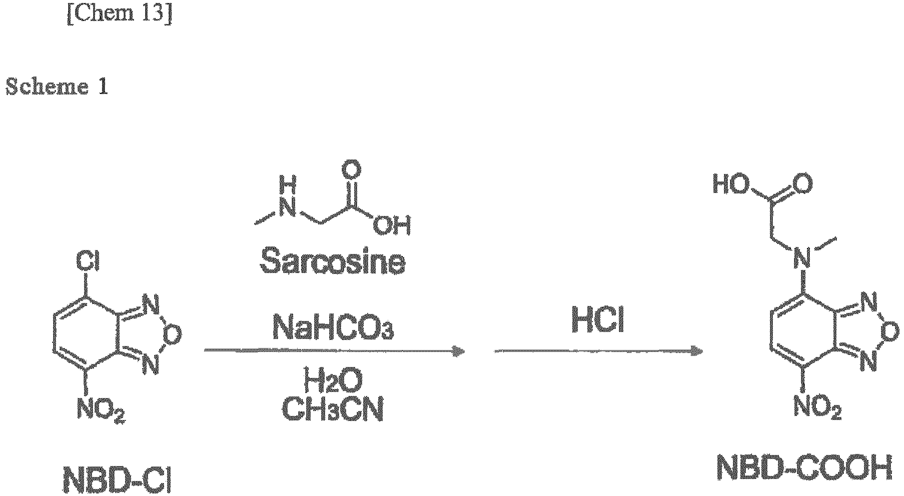

In a darkened room, N-methyl glycine (1.85 g, 20.8 mmol) was dissolved in a 0.52 M sodium bicarbonate aqueous solution (100 ml), and an acetonitrile solution (50 ml) of NBD-Cl (1.04 g, 5.23 mmol) was added thereto by dripping. The solution was stirred for 2 hours at room temperature then the acetonitrile was evaporated off under reduced pressure. The solution was made acidic by the addition of a 6 M aqueous hydrochloric acid solution (14 ml) then stirred for 30 minutes at room temperature. The precipitated solid was suction filtered, and dried under reduced pressure to obtain orange solid (1.35 g, quant).
1H NMR (400 MHz, DMSO-d6) δ 8.54 (d, J = 9.0 Hz, 1H), 6.47 (d, J = 9.2 Hz, 1H), 4.91 (s, 2H), 3.47 (s, 3H).

### (2) Synthesis of 2,5-dioxopyrrolidin-1-yl 2-[N- (4-nitrobenzo [c] [1,2,5] oxadiazol-7-yl)-N-methylamino] acetate (NBD-COOSu)

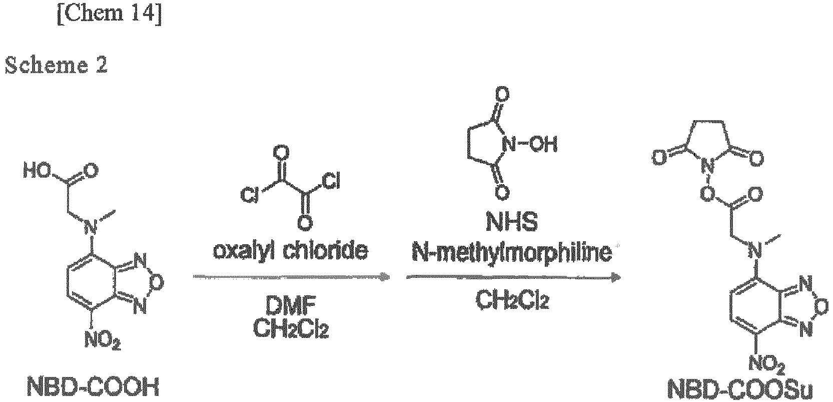

In a darkened room, the NBD-COOH (236 mg, 0.935 mmol) prepared in Example 1 was dissolved in methylene chloride (2 ml) and oxalyl chloride (1.1 ml) was added to the solution by dripping. N,N-dimethylformamide (5 µl) was added to the solution then stirred for 1 hour at room temperature. Thereafter, the solvent was evaporated off under reduced pressure and vacuum drying was carried out. The obtained residue was dissolved in methylene chloride (1 ml) and was dripped into a solution of methylene chloride (1 ml) in which N-hydroxysuccinimide (115.2 mg, 1.00 mmol) and N-methyl morpholine (0.1 ml) were dissolved. After stirring for one day, methylene chloride (50 ml) was added, and the solution was washed with water (20 ml) twice, with a saturated aqueous sodium bicarbonate solution (20 ml) twice, and with water (20 ml) once again and dried on sodium sulfate. After the sodium sulfate was filtered off, the solvent was evaporated off under reduced pressure to obtain an orange powder (206 mg, yield 63.2%).
1H NMR (500 MHz, CD 2 Cl 2) δ 8.50 (d, J = 8.7 Hz, 1 H), 6.32 (d, J = 8.8 Hz, 1H), 5.26 (s, 2H), 3.52 (s, 3H), 2. 84 (s, 4H).

### EXAMPLE 2: Fluorescence derivatization of amino acids using NBD-COOSu

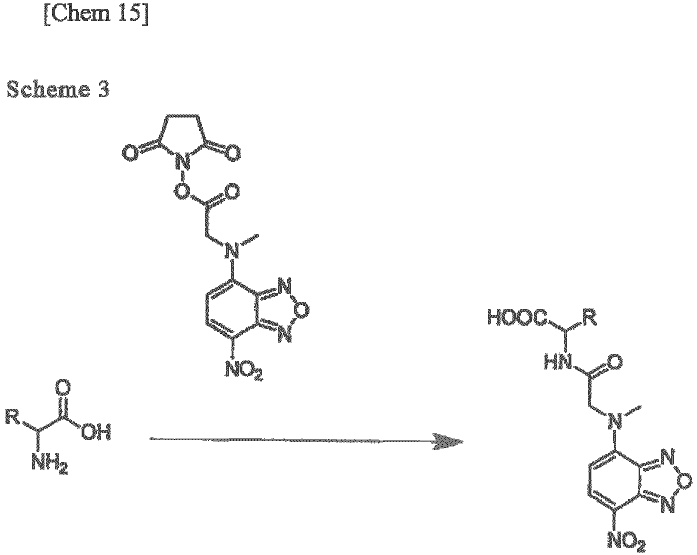

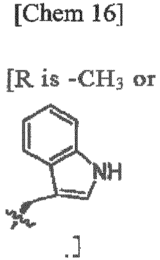

A 400 mM borate buffer (pH 8.0) was added to an aqueous tryptophan solution (10 µl) comprising both 20 pM of D-tryptophan and 80 pM L-tryptophan and an aqueous alanine solution (10 µl) comprising both 0.5 pM of D-alanine and 2 pM of L-alanine and a 40 mM NBD-COOSu acetonitrile solution (5 µl) was further added thereto and reacted at 60 °C for two minutes. Next, a 0.2% aqueous trifluoroacetic acid solution (75 µl) was added, the reaction was haltedto obtain amino acid derivatives (NBD-CO-Trp and NBD-CO-Ala).

### EXAMPLE 3: Separation by HPLC

HPLC analysis was performed on the amino acid derivatives obtained in Example 2. The column used for the HPLC analysis was an original Pirkle type column (JP Pat. No. 5977765) wherein KSAACSP-001R (1.5 mm i.d. × 250 mm) was used as the chiral solid phase, and acetonitrile : methanol (30:70) was used as the mobile phase. Detection was carried out using a fluorescence (excitation wavelength 470 nm, fluorescence wavelength 530 nm) and UV/VIS (measurement wavelength 470 nm) detector. HPLC analysis was performed on the aqueous alanine derivative solution. After the analysis, the column was washed (with methanol for 10 minutes, with ammonium formate for 10 minutes, then again with methanol for 20 minutes), stabilization was carried out for 40 minutes with the eluate, then HPLC analysis was performed on the aqueous tryptophan derivative solution. The chromatogram obtained by fluorescence detection is shown in FIG. 2B. Further, instead of fluorescence detection, detection was performed based on mass analysis. The chromatogram obtained by detection based on mass analysis is shown in FIG. 2C.

### Comparative Example 1: HPLC analysis in the case of NBD-F

10 µl of a 400 mM borate buffer solution (pH 8.0) was added to standard amino acid samples comprising proteinogenic amino acids including allo forms thereof and a 40 mM NBD-F solution (5 µl) was further added thereto and reacted at 60 °C for two minutes. Next 0.2% aqueous trifluoroacetic acid solution (75 µl) was added, the reaction was halted, and amino acid derivatives were obtained. HPLC was performed on the obtained amino acid derivatives with HPLC apparatus comprising a reverse phase column (monolithic ODS column, 0.53 mm i.d. × 1000 mm manufactured by Shiseido) set to 45 °C. Gradient elution was performed using an aqueous mobile phase comprising 5 to 35% MeCN, 0 to 20% THF, and 0.05% TFA at a flow rate of 25 µl/min. A chromatogram obtained by fluorescence detection is shown in FIG. 1. The intensity of tryptophan was extremely low showing that quantification was difficult.

### Comparative Example 2: chiral HPLC analysis in the case of NBD-F

10 µl of a 400 mM borate buffer solution (pH 8.0) was added to an aqueous tryptophan solution (10 µl) comprising both 20 pM of D-tryptophan and 80 pM L-tryptophan and an aqueous alanine solution (10 µl) comprising both 0.5 pM of D-alanine and 2 pM of L-alanine and a 40 mM NBD-F solution (5 µl) was further added thereto and reacted at 60 °C for two minutes. Next, a 0.2% aqueous trifluoroacetic acid solution (75 µl) was added, the reaction was halted, and amino acid derivatives (NBD-Trp and NBD-Ala) were obtained.

HPLC analysis was performed on the obtained amino acid derivatives in the same way as in Example 3. The chromatogram obtained by fluorescence detection is shown in FIG. 2A.

Comparative Example 2 shows the results of derivatizing alanine and tryptophan using conventional NBD-F then performing chiral HPLC on each of the derivatives. In the case of alanine, separate peaks can be observed for D-alanine and L-alanine. However, in the case of tryptophan, the peaks for D-tryptophan and L-tryptophan were extremely small and could not be quantified (FIG. 2). Although not intended to be limited by theory, it is believed that quenching occurs as a result of photoinduced electron transfer occurring between the NBD ring and the indole ring of tryptophan. Meanwhile, Example 3 shows the results of derivatizing alanine and tryptophan using NBD-COOSu then performing chiral HPLC on each derivative. In the case of both alanine and tryptophan, separate peaks could be observed for the D form and L form (2B). Moreover, the peaks matched the detection results of mass analysis. These results show that amino acid derivatization using NBD-COOSu is effective for separation and detection of D and L forms of amino acids.

## Claims

1. A compound according to the following formula: 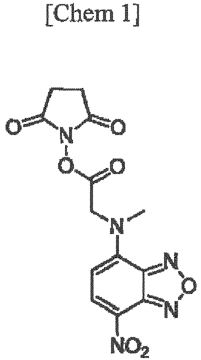 or a salt thereof.

2. A fluorescence derivatization reagent comprising a compound according to the following formula: 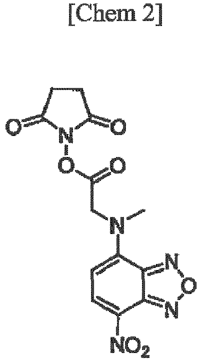 or a salt thereof.

3. A method for analyzing an amino group-containing compound in a sample comprising:
reacting the sample comprising the amino group-containing compound with a compound according to the following compound: 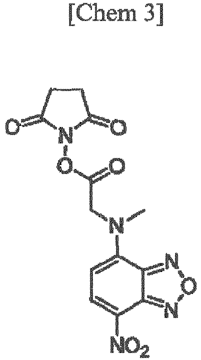 ; and
subjecting a reaction product to chromatography.

4. The method according to claim 3, wherein the method of analysis is a method of optically resolving optical isomers and the chromatography is chiral chromatography.

5. The method according to claims 3 or 4 wherein the amino group-containing compound is an amino acid.

6. A method for producing an amino acid derivative comprising:
reacting an amino acid with a compound according to the following formula:

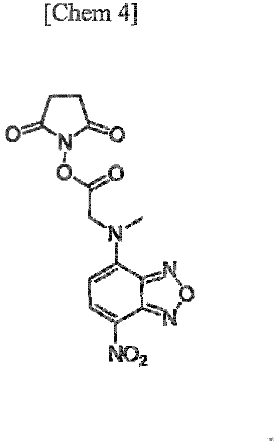

7. A compound according to the following formula: 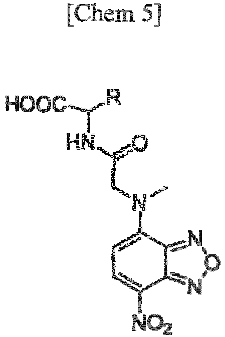 wherein R is an amino acid side chain.
